# EUROPEAN PATENT SPECIFICATION

(11) **EP 3 226 967 B1**
(45) Date of publication and mention of the grant of the patent: **29.04.2026**
(21) Application number: 15865084.6
(22) Date of filing: 04.12.2015
(51) Int. Cl.: A61N 1/36, A61N 2/00

(54) **SYSTEM FOR PREVENTATIVE MIGRAINE HEADACHE TREATMENT**
SYSTEM FÜR PRÄVENTIVE MIGRÄNEBEHANDLUNG
SYSTÈME DE TRAITEMENT PRÉVENTIF DE LA MIGRAINE

(30) Priority: 05.12.2014 US 201462088402 P
(43) Date of publication of application: 11.10.2017
(73) Proprietor: Aruene Corporation, Wilmington, DE 19808 (US)
(72) Inventor: GOADSBY, Peter J., San Francisco, CA 94158 (US); BHOLA, Ria, 10133 Tallinn (EE)
(74) Representative: Mathys & Squire
(86) International application number: PCT/US2015/064128
(87) International publication number: WO 2016/090333

(56) References cited:
- WO-A1-02/09811
- US-A1- 2005 234 286
- US-A1- 2006 047 316
- US-A1- 2010 228 075
- US-A1- 2013 137 918
- US-B1- 6 402 678
- US-B1- 6 402 678

## Description

### BACKGROUND OF THE INVENTION

### 1. Field of the Invention.

The present invention relates to the prophylactic treatment of neurological disorders, for example migraine episodes. Although embodiments make specific reference to the treatment and diagnosis of migraine episodes, sometimes called attacks (hereinafter "migraines"), with magnetic fields, embodiments of the present invention may have application to the prophylactic treatment of many neurological disorders, for example depression, Parkinson's' disease, stroke, chronic pain, tinnitus and epilepsy.

Many people suffer from migraine episodes. Migraine episodes can be debilitating and can affect patient quality of life and productivity. The migraine episode can debilitate a person, may last for up to seventy two hours, and may require medical attention in at least some instances. Although many people associate migraine episodes with headaches, a migraine episode can include four phases: a premonitory phase also referred to as "pre-headache"; an aura phase; a headache phase; and a postdrome phase. When present, the premonitory phase occurs prior to the migraine headache phase. The premonitory phase may be experienced for hours or even days before the migraine headache phase, and may include symptoms such as altered mood, irritability, depression or euphoria, fatigue, yawning, excessive sleepiness, craving for certain food, stiff muscles, constipation or diarrhea, increased urination, and other visceral symptoms. At least some people with migraines can experience the aura phase, which can occur after the prodrome phase and before the headache phase. The aura phase may include visual symptoms and non-visual symptoms. The visual symptoms may include flashes of lights or formations of dazzling lines, blurred or shimmering or cloudy vision, and tunnel vision. The non-visual symptoms of the aura phase may include auditory and/or olfactory hallucinations, temporary dysphasia, vertigo, tingling or numbness of the face and extremities, and hypersensitivity to touch. The headache phase can include one or more of many symptoms, for example pain, photophobia, phonophobia, osmophobia, blurred vision, nasal stuffiness, diarrhea, polyuria, pallor, sweating, localized edema of the scalp or face, scalp tenderness, and stiffness and tenderness of the neck, in at least some instances. The postdrome phase can last for hours, in some instances days, and symptoms may include one or more of fatigue, poor concentration, poor comprehension, and lowered intellect level in at least some instances. Due to the painful and debilitating nature of migraine episodes that occur in at least some instances, effective treatments are currently sought. In some migraine patients, the disorder can manifest on a daily basis, and when occurring on fifteen days or more a month can be called chronic migraine.

A successful approach to migraine treatment has been to employ a portable magnetic pulse system, in which the system can deliver a short duration magnetic single pulse onto the patient's brain. Although such systems have been effective in treating migraines, they have thus far been used only for acute treatment of patients at the onset of a migraine attack. Acute treatment refers to the treatment of an individual migraine attack to relieve sever head pain along with associated symptoms including sensitivity to light, sound, touch and smell. These attacks are sometimes preceded by a sensory aura and may include nausea in the later stages of the attack. Preventive (or prophylactic) treatments are given at regular intervals to reduce the onset and severity of migraine attacks. Preventive treatments are considered for patients that suffer frequent migraine attacks.

Previous studies and literature cite single pulse transcranial magnetic stimulation (sTMS) as a potential (short term) acute treatment of a specific migraine attack. A multicenter randomized controlled trial (eNeura) of 164 patients treated for at least 1 attack of migraine with aura with a handheld single transcranial magnetic stimulation (sTMS) device (n=82) or with sham stimulation (n=82) reported that pain-free rates 2 hours after stimulation were significantly better with sTMS(39% [32/82]) than with sham stimulation (22% [18/82]; p=0.018). Sustained pain-free response rates (with no recurrence and no rescue drug use) significantly favored sTMS at 24 hours (29% [24/82] versus 16% [13/82]; p=0.0405) and 48 hours (27% [22/82] versus 13% [11/82]; p=0.0327) after treatment. See, Lipton RB, Dodick DW, Silberstein SD et al.(2010) Single-pulse transcranial magnetic stimulation for acute treatment of migraine with aura: a randomized, double-blind, parallel-group, sham-controlled trial. Lancet Neurol 9(4): 373-80. In single pulse Transcranial Magnetic Stimulation (sTMS), the magnetic stimuli are delivered not more than every few seconds (usually longer than 5-10 seconds) and are not coupled with another pulse. The effects of sTMS are thought to be of short duration.

Repetitive Transcranial Magnetic Stimulation (rTMS) has been proposed for prophylactic migraine treatment and involves regularly repeated (rapid) trains of pulses (500-1000) delivered to a single site on the scalp and cause increased or decreased excitability of the cortical pathways. The effects depend upon the intensity and frequency of stimulation and the orientation of the coils on the head. rTMS has been studied a prophylactic treatment for depression. A few small studies of rTMS for migraine prevention have been published. Researchers have theorized the lasting effect of rTMS could have a preventive effect in migraine. The train of repetitive pulses produces longer-lasting effects which persist past the initial period of stimulation with the capability of inducing neuroplasticity. rTMS can increase or decrease the excitability of the corticospinal tract depending on the intensity of stimulation, coil orientation, and frequency. The mechanism of these effects is not clear, though it is widely believed to reflect changes in synaptic efficacy akin to long-term potentiation (LTP) and long-term depression (LTD).

The use of sTMS has not been previously proposed as a prophylactic treatment for headaches and other migraine manifestations.

Therefore, a need exists for improved methods and apparatus for treatment of migraines and other neurological conditions. Ideally, such improved methods and apparatus will be able to reduce or prevent the occurrence of migraines and neurological conditions when used at times prior to onset and/or occurrence of a migraine attack.

### 2. Description of the Background Art.

Published Patent Applications and Patents that may be relevant to aspects of the present application include: 2001/0051819; 2001/0056290; 2002/0002390; 2003/0028072; 2003/0088290; 2003/0120324; 2004/0153129; 2004/0181115; 2004/0249422; 2006/0047316; 2006/0205993; 2006/0224216; 2010/0228075; U.S. Pat. Nos. 6,402,678; 7,223,234; and 7,294,101.
US 2010/0228075 describes a treatment apparatus configured to treat a patient for a neurological disorder comprises a patient interface configured for the patient to enter data for an assessment of his or her subjective sensations, for example pain symptoms associated with migraines. The subjective patient sensation data may comprise at least one of a patient symptom of the neurological condition or a patient trigger of the neurological condition. For example the subjective patient sensations may comprise symptoms that correspond to migraine such as aura symptoms. The treating physician can view the subjective patient data, and diagnose and treat the patient with a treatment plan by writing a treatment plan to a storage device, for example treatment instructions written to a smart card. The storage device can be delivered to the treatment apparatus such that the patient can be treated in response to physician's treatment plan.
US 6,402,678 describes a means and method for the treatment of migraine headaches. Patients who have migraine headaches typically have a band of excited brain neurons that are a precursor of the headache. By placing an intense alternating magnetic field onto a certain region of the brain, an electrical current can be generated in the cerebral cortex that can depolarize these excited brain neurons. The device can be placed in some headgear such as a bicycle helmet in order to place the magnetic field at the correct location relative to the patient's cerebral cortex.
US 2013/0137918 describes devices and methods for modulating the electrical activity of a brain in a targeted manner using a weak magnetic field, generally field with a strength of less than about 100 Gauss. The magnetic field is varied over time in a periodic manner, generally with a frequency tuned to specifically affect one of the intrinsic "Low Field Magnetic Stimulation" devices and methods describes herein modulate the electrical activity of a brain without requiring medication. Methods and devices described herein gently "tune" the brain and affect mood, focus and cognition of human subjects.

### BRIEF SUMMARY OF THE INVENTION

The present invention is set out in the appended claims. The present disclosure relates to the prophylactic treatment of migraine episodes and other neurological disorders. Although embodiments make specific reference to the prophylactic treatment of migraine episodes with magnetic fields, embodiments of the present disclosure will have application to the diagnosis and treatment of many neurological disorders, for example depression, Parkinson's disease, stroke, chronic pain, tinnitus and epilepsy.

A treatment apparatus may comprise circuitry configured to generate a magnetic field to treat patients prior to the onset or occurrence of migraines, usually on a periodic schedule selected to inhibit the onset of a migraine headache in a patient, as described more specifically below and in the claims. The treatment apparatus may comprise a patient interface, for example a touch screen display, in which the patient interface is configured for the patient to enter subjective patient data for an assessment of his or her subjective sensations, for example pain and other symptoms associated with migraines. The patient treatment apparatus comprising a patient interface configured for the patient to enter particular treatment parameters, including power, duration, and pulse characteristics. Often the apparatus will also allow the patient to enter his or her own subjective patient data such that a treating physician can more accurately diagnose and treat the patient having the migraine or other neurological disorder.

Migraine headaches and other manifestations are inhibited by positioning an electromagnetic field generator adjacent a patient's head. An electromagnetic field from the electromagnetic field generator with an intensity in the range from 0.85 Tesla to 1.3 Tesla is directed at the patient's head for a time in the ranges from 0.001 second to 1 second. The treatments are repeated in a time pattern selected to inhibit the onset of migraine headaches and other manifestations in the patient.

The time pattern may comprise repeating steps (a) and (b) no more often than every 3 hours and no less often that every 24 hours, usually repeating steps (a) and (b) no more often than every 6 hours and no less often that every 24 hours, more usually repeating steps (a) and (b) no more often than every 12 hours and no less often that every 24 hours, and typically repeating steps (a) and (b) daily.

In preferred embodiments steps (a) and (b) are repeated regardless of the patient's symptoms. In other embodiments, the methods may further comprise directing a therapeutic electromagnetic field toward the patient's head whenever the patient feels the onset of a migraine headache in addition to repeating steps (a) and (b). The therapeutic electromagnetic field directed at the patient's head whenever the patient feels the onset of a migraine headache will typically have an intensity in the range from 0.85 Tesla to 1.3 Tesla for a time in the ranges from 0.001 second to 1 second. In most cases, the patient self-administers the prophylactic electromagnetic field, typically by holding the electromagnetic field generator while delivering the prophylactic electromagnetic field.

The frequency of severe and excruciating migraine headaches experienced by the patients using the prophylactic methods of the present disclosure is typically reduced by at least 25% when compared to the frequency of migraine headaches experienced by the patient while not practicing the present disclosure. Often, the frequency is reduced by at least 50%, and sometimes the frequency is reduced by at least 60%, or more.

### BRIEF DESCRIPTION OF THE DRAWINGS

FIG. 1A shows a patient treating herself to inhibit migraine onset with a hand held treatment device, in accordance with embodiments .
FIG. 1B shows the user interface of the hand held treatment device as in FIG. 1A.
FIG. 1C shows a schematic illustration of the components of the hand held treatment device as in FIGS. 1A and 1B.
FIG. 1D shows a computer program embodied on a tangible medium comprising instructions to permit and control treatment of the patient with the at least one processor of FIGS. 1A and 1B.
FIGS. 2 and 3 are bar graphs illustrating data generated by experiments described in the Experimental section below.

### DETAILED DESCRIPTION

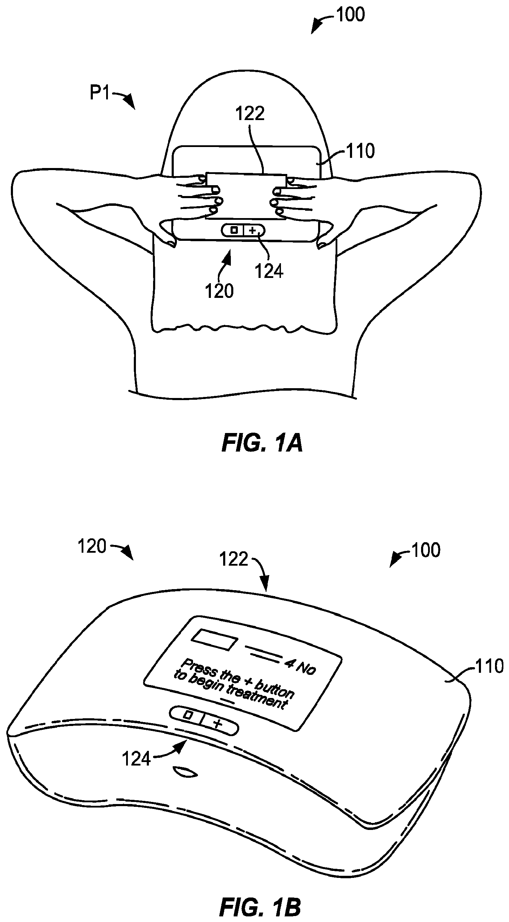

FIG. 1A shows a first patient P1 treating herself in order to inhibit the onset of a migraine headache or other neurological disorder with a hand held treatment device 110. The hand held treatment device may comprise a component of a treatment system 100. For treatment, the patient can place treatment device 110 near the back of the head and neck. The treatment device 110 generates a magnetic field to treat the patient. Treatment device 110 includes a patient interface 120. Patient interface 120 comprises a display 122 and at least one button 124. Display 122 may comprise a touch screen display. At least one button 124 may comprise a plurality of buttons.

FIG. 1B shows hand held treatment device 110 and patient interface 120 as in FIG. 1A. Display 120 may comprise instructions for the patient, for example "Press the + button to begin treatment". The display can show menus for the patient to select treatment or entry of patient data. Although the display shown may comprise a curved surface with a centrally located display, many configurations of the handheld treatment device 110 can be used. For example, the surface with the display may be flat, and the other surfaces curved. Alternatively or in combination, the treatment device may comprise a substantially rectangular geometry with many flat surfaces and corners with right angles.

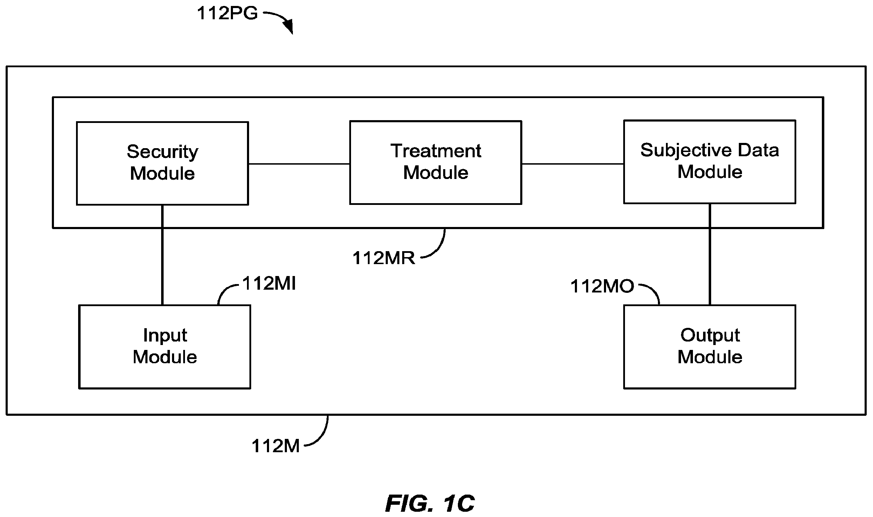

FIG. 1C shows a schematic illustration of the components of the hand held treatment device 110 as in FIGS. 1A and 1B. Many components of hand held treatment device 110 can be similar to components described in Pub. No. US 2006/0047316 , which application includes subject matter that may be suitable for combination with the teachings described herein in accordance with some embodiments . Patient treatment device 110 comprises a processor 112, which may comprise one or more processors, for example a distributed processor system. Processor 112 comprises a tangible medium such as a memory 112M. The memory may comprise volatile memory such as random access memory and non-volatile memory such as flash RAM. The memory 112M of processor 112 may comprise a configuration file, or "config." file, that includes parameters for the system to operate and deliver treatment to the patient, for example calibration and control voltage parameters. The processor 112 can be coupled to many additional components of the treatment device. The processor 112 comprises at least one processor, for example a single processor with instructions for treatment, and may comprise an additional processor, for example a display processor coupled to a touch screen display to control the acquisition of data from the patient.

Processor 112 can be coupled to a patient specific module (hereinafter "PSM"), for example a known subscriber identity module (hereinafter "SIM"). The PSM may also comprise a known smart card with patient treatment information. For example SIM 124 may comprise a smart card configured to control at least some aspects of the patient treatment, for example the number of pulses available for the patient over a specific time period. SIM 124 may comprise additional treatment parameters such as the maximum number of pulses per unit time that the patient can deliver. SIM card 124 may also comprise instructions for a treatment plan, for example treatment commands. Processor 112 can be configured to reduce the number of treatments available for patient P1 that are stored on SIM 114 in response to delivery of a treatment to the patient. Processor 112 may comprise instructions to treat the patient in response to parameters stored on the SIM 124, for example the number of treatments. The SIM 124 may comprise a card that is inserted into the treatment device. SIM 124 may comprise a number keyed to the treatment device. For example, patient treatment device 110 may comprise a serial number written to non-volatile memory and SIM 124 can be keyed to the serial number.

The treatment parameters written to the SIM card may comprise a treatment plan. The treatment plan will typically provide for a desired prophylactic treatment regimen within the time intervals set forth above, e.g. typically being once a day but optionally more or less often. In some cases, the treatment plan and programming of the device may specify a maximum number of treatments over a period of time, for example no more than one treatment every six hours. The treatment plan may and programming may also specify minimum number of treatments during a time interval, for example no less than one treatment per day. The treatment plan may also comprise a number of pulses to be delivered with each prophylactic treatment.

Processor 112 can optionally be coupled to a patient interface 120. The patient interface 120 may comprise many known interface components, for example known displays, touch screens, buttons and buzzers. An input device of interface 120 may comprise a touch screen 122. The input device may comprise many known input devices such as pointing devices, keyboards and touch screen 122. Patient interface 122 comprises a treat button 124 for the patient to initiate treatment. However, the treat button can provide additional input, for example for the patient to enter data. Patient interface 120 comprises an alarm, for example a buzzer 126, configured to alert the patient. Buzzer 126 can alert the patient when it is time for the patient to enter additional information into the patient journal. Buzzer 126 can also alert the patient that it is time for a treatment with the magnetic field.

Processor 112 can be coupled to communication circuitry 116. Communication circuitry 116 may comprise a USB port on treatment device. Communication circuitry 116 may comprise wireless communication circuitry configured to communicate with a wireless communication protocol, for example a Bluetooth^{®} protocol. Communication circuitry 116 can upload patient usage and data to a remote server where the patient data can be stored for review by a physician. Communication circuitry 116 can also download treatment related parameters, for example parameters for the configuration file. The treatment related parameters of the configuration file may be stored on SIM 114.

The patient specific module may comprise a processor, for example a processor of a smart card, such that the tangible medium of the smart card and the processor of the smart card comprise the at least one processor of the treatment device. The instructions for treatment can be embodied in tangible medium of the at least one processor, in which a computer program comprising instructions for patient treatment is embodied on the tangible medium.

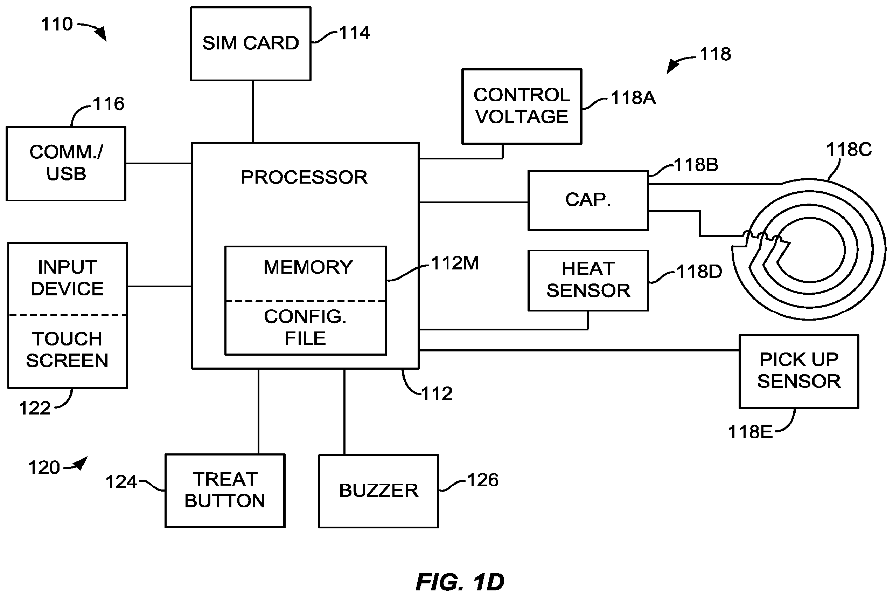

FIG. 1D shows a computer program 112PG embodied on tangible medium 112M comprising instructions to permit and control prophylactic treatment of the patient with the at least one processor of FIGS. 1A, 1B and 1C. Computer program 112PG comprises an input routine 112MI, and output routine 112MO and a run routine 112MR. Input routine 112MR may comprise an input module operatively coupled to a source of data, for example at least one of the treatment and patient data of the smart card or the subjective input data of the patient interface. Run routine 112MR may comprise, for example, a security module, a treatment module and a subjective data module to collect the subjective data from the patient. The security module may comprise instructions configured to process the patient specific identifier and the device identifier and encryption codes of the smart card. The treatment module may comprise instructions configured to determine the treatment parameters to the treatment circuitry in response the patient treatment parameters from the patient specific module and the configuration file. The subjective data module can be configured to ask questions of the patient as described above. Each of the security module, the treatment module and the subjective data module can be operatively coupled to the output module to output relevant information to the user and physician as appropriate. For example, the security module can indicate when the patient specific module is read correctly when inserted into the receptacle. The treatment module can output treatment parameters to the circuitry for treatment, and the subjective data module can output data to another computer such that the subjective data can be sent to the treating physician.

### EXPERIMENTAL

### Method:

Patients were instructed to treat daily with 2 sequential pulses, wait 15 minutes and repeat two sequential pulses; each morning and evening. If needed patients could increase to 3 and subsequently, 4 sequential pulses each morning and evening. Patients also had the option to treat at the midday time point. For prevention, the daily number of pulses ranged from 8 to 24 pulses per day over a 3-month period. Additionally, 4 pulses could be used to treat acute migraine attacks as needed during this period.

Twenty-seven (27) patients with frequent migraine (more than 15 days per month) were prescribed daily use of single pulse TMS (sTMS). The patients headache patterns were assessed at baseline (before they began daily use of sTMS), at 6 weeks after using sTMS preventatively (every morning and evening) and again after using the device preventatively for 12 weeks. Patients were asked to keep a headache diary and provide the number of days they experienced a migraine headache attack during the past month at each time point (baseline, 6 weeks and 12 weeks). They were also asked to estimate the level of pain during migraine attack.

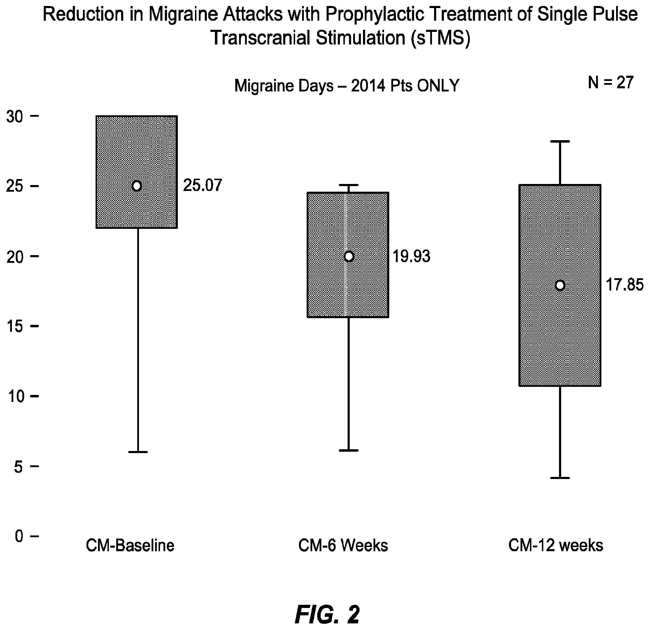

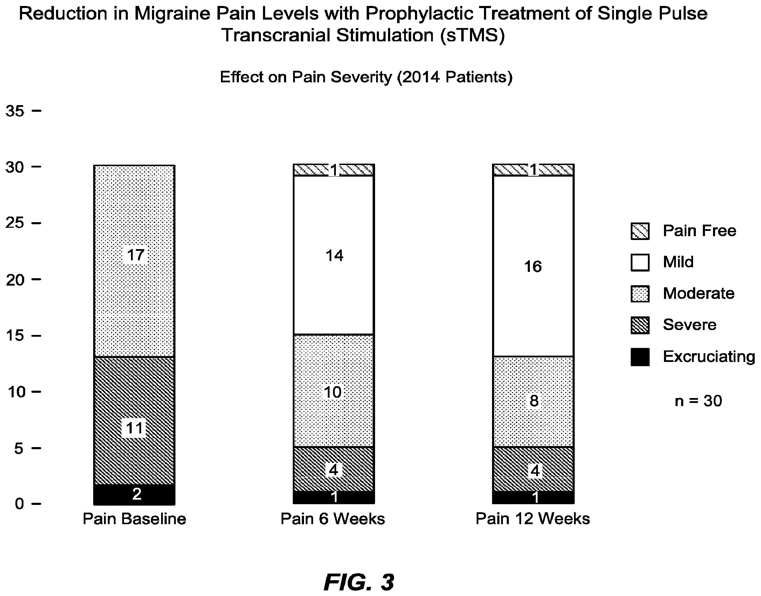

### The Results are shown in FIGS. 2 and 3.

FIG. 2 shows the reduction in migraine attack days over the 12 week period when using sTMS preventatively. The mean number of attack (days) reported at baseline (before sTMS use) was 25 over the last 30 days. After 6 weeks of preventive sTMS use, the mean number of attack (days) dropped to 20 over the prior 30 days. After 12 weeks of use, the mean number of migraine attack (days) dropped to 18 days over the previous 30 days. The figure shows a mean reduction of 7 migraine attack (days) per month after 12 weeks of preventative use. This is a 29% reduction in the number of migraine attack days per month. This reduction is considered clinically meaningful and consistent with migraine prevention medications but without the medication related side effects.

In addition to the reduction in number of migraine attack days, patients reported a significant reduction in attack pain levels as shown in FIG. 3. The number of patients reporting excruciating or severe pain with each migraine attack dropped from 13 at baseline to 5 after 6 weeks of sTMS preventive use. This represents 61% reduction in pain severity per attack. The reduction in severity was maintained at 12 weeks. The number of patients reporting moderate pain levels at base line was 17 with no patients reporting mild pain or being pain free. At week 6, the number of patients reporting moderate pain dropped to 10 with 14 patients reporting mild pain and one patient being pain free for the entire 30 day period. At week 12, the number of patients reporting mild pain dropped further to 8 from 10, and the number reporting mild pain (as opposed to moderate pain) increased to 16. Pain severity is directly correlated to migraine related disability. A reduction in pain severity is a clinically meaningful improvement in migraine disability.

The methods of the present disclosure are not limited to the use of any particular apparatus for generating and delivering the prophylactic magnetic field to the patient.

## Claims

1. A system for inhibiting onset of a migraine headache of a patient, the system comprising:
an electromagnetic field generator (118C) configured to be held adjacent the patient's head and to direct a prophylactic electromagnetic field with an intensity in the range from 0.85 Tesla to 1.3 Tesla for a time in the ranges from 0.001 second to 1 second from the electromagnetic field generator toward the patient's head; and
control means (112) for:
(a) providing an alert to the patient that it is time for treatment with the magnetic field; and
(b) causing the electromagnetic field generator (118C) to direct a prophylactic electromagnetic field with an intensity in the range from 0.85 Tesla to 1.3 Tesla for a time in the ranges from 0.001 second to 1 second from the electromagnetic field generator toward the patient's head, the control means being configured to repeat (b) according to a time pattern, wherein the time pattern comprises repeating (b) no more often than every 3 hours and no less often that every 24 hours.

2. A system as in claim 1, wherein the time pattern comprises repeating (b) no more often than every 6 hours and no less often that every 24 hours.

3. A system as in claim 1, wherein the time pattern comprises repeating (b) no more often than every 12 hours and no less often that every 24 hours.

4. A system as in claim 1, wherein the time pattern comprises repeating (b) daily.

## Patentansprüche

1. System zum Hemmen des Auftretens von Migränekopfschmerzen bei einem Patienten, wobei das System Folgendes umfasst:
einen elektromagnetischen Feldgenerator (118C), der dazu konfiguriert ist, in der Nähe des Kopfes des Patienten gehalten zu werden und ein prophylaktisches elektromagnetisches Feld mit einer Intensität in dem Bereich von 0,85 Tesla bis 1,3 Tesla für eine Zeit in den Bereichen von 0,001 Sekunde bis 1 Sekunde von dem elektromagnetischen Feldgenerator in Richtung des Kopfes des Patienten zu richten; und
ein Steuermittel (112) für Folgendes:
(a) Bereitstellen eines Warntons für den Patienten, dass es Zeit für die Behandlung mit dem magnetischen Feld ist; und
(b) Bewirken, dass der elektromagnetische Feldgenerator (118C) ein prophylaktisches elektromagnetisches Feld mit einer Intensität in dem Bereich von 0,85 Tesla bis 1,3 Tesla für eine Zeit in den Bereichen von 0,001 Sekunde bis 1 Sekunde von dem elektromagnetischen Feldgenerator in Richtung des Kopfes des Patienten richtet, wobei das Steuermittel dazu konfiguriert ist, (b) gemäß einem Zeitmuster zu wiederholen, wobei das Zeitmuster das Wiederholen von (b) nicht häufiger als alle 3 Stunden und nicht seltener als alle 24 Stunden umfasst.

2. System nach Anspruch 1, wobei das Zeitmuster das Wiederholen von (b) nicht häufiger als alle 6 Stunden und nicht seltener als alle 24 Stunden umfasst.

3. System nach Anspruch 1, wobei das Zeitmuster das Wiederholen von (b) nicht häufiger als alle 12 Stunden und nicht seltener als alle 24 Stunden umfasst.

4. System nach Anspruch 1, wobei das Zeitmuster das tägliche Wiederholen von (b) umfasst.

## Revendications

1. Système conçu pour inhiber l'apparition d'une céphalée migraineuse chez un patient, le système comprenant :
un générateur de champ électromagnétique (118C) configuré pour être tenu à côté de la tête du patient et pour diriger un champ électromagnétique prophylactique ayant une intensité dans la plage de 0,85 Tesla à 1,3 Tesla pendant une période dans la plage de 0,001 seconde à 1 seconde du générateur de champ électromagnétique vers la tête du patient ; et
un moyen de commande (112) conçu pour :
(a) fournir une alerte au patient indiquant que le moment est venu pour un traitement avec le champ magnétique ; et
(b) amener le générateur de champ électromagnétique (118C) à diriger un champ électromagnétique prophylactique ayant une intensité dans la plage de 0,85 Tesla à 1,3 Tesla pendant une période dans la plage de 0,001 seconde à 1 seconde du générateur de champ électromagnétique vers la tête du patient, le moyen de commande étant configuré pour répéter l'étape (b) selon un schéma temporel, le schéma temporel comprenant une répétition de l'étape (b) pas plus souvent que toutes les 3 heures et pas moins souvent que toutes les 24 heures.

2. Système selon la revendication 1, dans lequel le schéma temporel comprend une répétition de l'étape (b) pas plus souvent que toutes les 6 heures et pas moins souvent que toutes les 24 heures.

3. Système selon la revendication 1, dans lequel le schéma temporel comprend une répétition de l'étape (b) pas plus souvent que toutes les 12 heures et pas moins souvent que toutes les 24 heures.

4. Système selon la revendication 1, dans lequel le schéma temporel comprend une répétition quotidienne de l'étape (b).
